# EUROPEAN PATENT SPECIFICATION

(11) **EP 1 379 286 B1**
(45) Date of publication and mention of the grant of the patent: **21.06.2006**
(21) Application number: 02723855.9
(22) Date of filing: 12.04.2002
(51) Int. Cl.: A61L 9/01

(54) **COMPOSITION AND METHOD FOR REDUCING ODOR AND DISINFECTING**
ZUSAMMENSETZUNG UND VERFAHREN ZUR REDUZIERUNG VON GERUCH UND ZUR DESINFEKTION
COMPOSITION ET PROCEDE SERVANT A ATTENUER LES ODEURS ET A DESINFECTER

(30) Priority: 16.04.2001 US 284072 P
(43) Date of publication of application: 14.01.2004
(73) Proprietor: JohnsonDiversey, Inc., Sturtevant, WI 53177-0902 (US)
(72) Inventor: HERNANDEZ, Pablo, M., Waukegan, IL 60085 (US); KRON, Ryan, E., Racine, WI 53402 (US); WADA, Mari, Tokyo, Tokyo 146-0085 (JP)
(74) Representative: Ruschke, Hans Edvard
(86) International application number: PCT/US2002/011759
(87) International publication number: WO 2002/083189

(56) References cited:
- GB-A- 2 346 900

## Description

### TECHNICAL FIELD

The present invention relates to aqueous compositions used for applying to surfaces of objects to reduce malodor carried by the object. These compositions are alcohol-free and contain small amounts of aldehyde-containing odor counteractant compositions as the main, malodor counteractant. They may also contain small amounts of fragrance. In addition, the compositions contain selected quaternary amine disinfectant compounds. The compositions are generally applied by spraying a small amount of the material onto the surface to be treated, such as tables, counters, fixtures, carpets, curtains, upholstery, bedspread and the like.

### BACKGROUND OF THE INVENTION

Living environments can from time-to-time become contaminated with unpleasant odors that are deposited on and adsorbed or absorbed into various components of the environment. When this occurs outdoors, it is not so objectionable, but when it occurs indoors, it can become objectionable, particularly to certain people who are sensitive to these odors. Cooking odors commonly occur in kitchens and can occasionally be objectionable and linger for long periods of time. Pet odors are common malodors that occur when pets are permitted in living areas. Dogs and cats are common household pets and their presence can cause objectionable odors to be present in the living space even when the animals are not present. Pet urine, particularly cat urine, is a particularly objectionable and troublesome odor to remove. Another particularly pervasive and common odor is that of tobacco smoke. When tobacco smoke has been present in a living space, many objects in the room retain the odor of the tobacco smoke and it is very difficult to remove.

Accordingly, a number of products have appeared in the marketplace to deal with this problem. They tend to be of two types: Either they mask the odor by providing a stronger, more pleasing odor, or they counteract the odor by chemically reacting with the malodor molecules and changing their fragrance or by creating a molecule with increased vapor pressure so that the new molecule does not change phase and vaporize into the atmosphere so easily or by some other mechanism.

### SUMMARY OF THE INVENTION

The environment contains many molecules that can be detected by the human olfactory senses. Generally, they are not offensive and, if so, only for a brief time. However, it has been found desirable on some occasions to reduce the detectability of certain odor molecules in the environment. Generally, this is done by filtering the air or by masking the presence of the odor molecules by adding other molecules that have a more pleasant smell and are preferentially detected by the olfactory senses. Many aerosol dispensed room air fresheners work in this way. However, it is not always desirable to merely mask the odor by adding additional detectable molecules. This often requires dispensing a large amount of material into the environment which may also be objectionable. The second fragrance may not mask the odors sufficiently and they may lose their effectiveness after a short period. More recently, compositions have been developed that do not merely"mask"the offensive odor with another odor, but which react with the key components of the offensive odors and make them less detectable, such as by increasing the vapor pressure of the offensive odors or some similar mechanism.

GB-A-2 346 900 describes sprayable deodorizing compositions useful for treating fibrous substrates, especially textiles and/or garments, and comprising a detersive surfactant, preferably at least one nonionic surfactant; an organic solvent; a deodorant constituent and water as well as optionally one or more constituents selected from chelating agents, light stabilizers, fragrances, fragrance solubilizers, thickening agents, hydrotropes, pH adjusting agents, pH buffers, builders, further non-aqueous solvents, optical brighteners, enzymes, preservatives, corrosion inhibitors and antistatic agents. Preferred examples of the deodorant constituents described therein are mixtures comprising at least one first aldehyde and at least one second aldehyde in a weight ratio of said first aldehyde to said second aldehyde of from 20 to 80 to 80 to 20. Preferred examples of usable organic solvents are at least partially water-miscible alcohols, glycols and glycol ethers. Furthermore it describes a process for deodorizing garments, textiles, upolstery and the like by applying an odor-diminishing effective amount of the composition described therein.

The present inventors have found that prior art masking compositions are unsatisfactory to reduce the malodor impression of certain odors, such as tobacco smoke, for any substantial time. Moreover, even the more superior counteractant compositions may not be able to be formulated into satisfactory concentrated compositions to enable their convenient shipping and subsequent on-site dilution or have the ability to counteract odors for a substantial period of time.

Therefore, the objective of the present invention is to find a malodor reducing method and composition which overcome certain of the deficiencies of the prior art methods and compositions.

Surprisingly it has been found that the above objective can be accomplished with compositions which comprise an odor counteractant component, a solubilizing agent for the odor counteractant component, a cosolvent for the odor counteractant component and a compatible quaternary amine disinfectant, each in specific amounts, together with an aqueous vehicle as the remainder. And the use of such composition in concentrated or diluted form in a method of counter acting odors of odor bearing in animate materials.

Subject-matter of the present invention is according to a first aspect a clear, stable, acidic, alcohol-free concentrate for reducing the odor of odor-bearing inanimate materials when diluted from 25 to 150 times with water, the concentrate comprising
(a) from 10 to 50 percent by weight of the total composition of an aldehyde-based odor counteractant component;
(b) from 25 to 60 percent by weight of the total composition of a solubilizing agent for the odor counteractant component;
(c) from 2 to 20 percent by weight of the total composition of a co-solvent for the odor counteractant component; and
(d) from 2 to 20 percent by weight of the total composition of a compatible quaternary amine disinfectant which does not degrade the performance of the odor counteractant component selected from the group consisting of N-alkyl, N-dimethylammonium chloride and didecyl dimethylammonium chloride, or mixtures thereof.
(e) the remainder being an aqueous vehicle.

According to further preferred embodiments the above concentrate additionally comprises an acidic pH control agent, a fragrance component and/or a preservative.

The odor counteractant component preferably comprises a combination of aldehyde compounds comprising
at least one first aldehyde selected from the group consisting of acyclic and non-terpenic aliphatic aldehydes, non-terpenic alicyclic aldehydes, terpenic aldehydes, aliphatic aldehydes substituted by an aromatic group, and bifunctional aldehydes and at least one second aldehyde selected from the group consisting of aldehydes possessing a non-aromatic unsaturation carried by the carbon in the alpha position of the aldehyde function, aldehydes possessing an unsaturation in the alpha position of the aldehyde function conjugated with an aromatic ring, and aldehydes of which the function is carried by an aromatic ring.

A particularly preferred odor counteractant component comprises at least one aldehyde compound having a vapor pressure no greater than about 4 Pascals at 25 degrees centigrade.

The preferred solubilizing agent used in the above concentrate is alkyloxypolyethyleneoxyethanol.

A preferred example of the co-solvent usable in the concentrate of the present invention is a glycol ether.

Subject-matter of the present invention is according to a second aspect a liquid compositon for applying to the surface of an odor-bearing inanimate material without further dilution, the composition comprising
(a) from 0.1 to 1.0 percent by weight of the total composition of an aldehyde-based odor counteractant component;
(b) from 0.3 to 0.8 percent by weight of the total composition of a solubilizing agent for the counteractant component;
(c) from 0.03 to 0.3 percent by weight of the total composition of a co-solvent for the counteractant component; and
(d) from 0.05 to 5.0 percent by weight of a compatible quaternary amine disinfectant which does not degrade the performance of the odor counteractant component selected from the group consisting of N-alkyl, N-dimethyl ammonium chloride and didecyl dimethylammonium chloride, or mixtures thereof.
(e) the remainder being an aqueous vehicle.

A preferred example of the cosolvents usable in this liquid composition is a glycol ether.

Subject-matter of the present invention is according to a third aspect a method of counteracting odors which is characterized in that it comprises the following steps
(a) preparing a clear, stable, acidic, alcohol-free concentrate as defined above;
(b) diluting the composition to at least about 25 times with water; and
(c) applying the diluted composition onto an odor-bearing surface.

In the above method the composition is preferably diluted between 25 to 150 times with water.

The compositions of the present invention provide superior malador suppression, for example of tobacco smoke, for an extended period of time following application of the composition. Moreover, the compositions of the present invention are able to be formulated as stable, clear concentrate emulsions which can be diluted at the point of use to stable, clear malodor-reducing compositions which also have sanitizing, disinfecting or biocidal properties. The compositions of the invention can be prepared in concentrated form for commercial transport and then diluted for use with water. It has been found, that the odor counteractant compositions of the present invention can be prepared in relatively concentrated form without the need for low molecular weight alcohols which are normally necessary for solubilizing certain fragrance compositions used for malodor reduction.

The preferred odor counteractant components are aldehyde-containing compositions which may also optionally include some fragrance component. Suitable counteractant compositions are described in US patents 5,795,566 and 4,840,792. The counteractant component is present in the concentrated form of the composition in amounts from about 10% to about 50% by weight of the composition and most preferably about 25% by weight of the composition. One part of this concentrated composition can be diluted with about 25 parts or more, preferably from about 25 to 150 parts and most preferably about 75 parts by weight water so that the counteractant component is present in the ready to use form in an amount of from about 0. 1 % to about 1% by weight of the total composition.

The counteractant compositions described above are generally not readily watersoluble so that other ingredients are needed to help solubilize them in an aqueous vehicle. The term "solubilize" as used herein means the formation of a stable, homogeneous liquid composition whether in emulsion, microemulsion or solution form. Therefore, the composition also includes a solubilizing agent and a co-solvent to assist in solubilizing the counteractant component in the aqueous vehicle.

Generally solubilizing agents alone, typically surfactants alone or in combination, do not provide for the formation of stable, gel-free liquids that can provide a clear, transparent, dilutable liquids in solution or emulsion form. The co-solvent works in combination with the solubilizing agent to enable the counteractant component to be solubilized in water at the relatively high concentrations needed for providing dilutable, commercial quality concentrates without gelling.

The above ingredients are combined in an aqueous vehicle, generally water. Other ingredients may optionally be added to the composition. For example an acid may be added to control the pH of the composition to be slightly acidic so that the counteractant will more readily react with amine-based malodor molecules. Citric acid is useful to control the pH of the composition. A preservative may also be added to keep bacteria or molds from growing during storage or after application. The preservative should be chosen so that it does not react with the counteractant component and reduce its effectiveness.

The biocidal component is added to the counteractant compositions of the present invention are selected from the group consisting of N-alkyl, N-dimethylammonium chloride and didecyl dimethylammonium chloride or mixtures thereof which have been found to be effective disinfectants which do not significantly reduce the efficacy of the aldehyde counteractant composition.

In another embodiment, the present invention relates to dilute liquid compositions for reducing the odor of odor-bearing, inanimate materials when applied to the surface of the materials. These compositions comprise from about 0.1 to 1.0 percent by weight of the total composition of an odor counteractant component, from about 0.3 to 0.8 percent by weight of the total composition of a solubilizing agent for the counteractant component, from about 0.03 to about 0.3 percent by weight of the total composition of a co-solvent for the counteractant component and from about 0.05 to 5.0 percent by weight of a compatible quaternary amine disinfectant selected from the group consisting of N-alkyl, N-dimethylammonium chloride and didecyl dimethylammonium chloride or mixtures thereof, which do not degrade the performance of the odor counteractant component, the remainder being an aqueous vehicle.

In yet another embodiment, the present invention relates to method for reducing the odor of odor-bearing, inanimate materials by applying a counteractant composition to the surface of the odor-bearing material wherein the method comprises preparing a dilutable, liquid composition comprising from about 10 to 50 percent by weight of the total composition of an odor counteractant component, from about 25 to 60 percent by weight of the total composition of a solubilizing agent for the counteractant component, from about 2 to about 20 percent by weight of the total composition of a co-solvent for the counteractant component, and from about 0.05 to 5.0 percent by weight of a compatible quaternary amine disinfectant selected from the group consisting of N-alkyl, N-dimethylammonium chloride and didecyl dimethylammonium chloride or mixtures thereof, which do not degrade the performance of the odor counteractant component, the remainder being an aqueous vehicle, and diluting the composition at least about 25 times with water and applying the diluted composition onto an odor-bearing surface.

In yet another embodiment, the present invention comprises a dilutable, disinfecting and odor counteracting composition comprising from about 10 to 50 percent by weight of the total composition of an odor counteractant component, from about 25 to 60 percent of the total composition of a solubilizing agent for the counteractant component, from about 3 to about 15 percent by weight of the total composition of a co-solvent for the odor counteractant component, and from about 3 to about 15 percent by weight quaternary amine disinfectant selected from the group consisting of N-dimethylammonium chloride and didecyl dimethylammonium chloride or mixtures thereof, which do not react with or otherwise significantly reduce the effect of the counteractant composition with the remainder being an aqueous vehicle and the method of using such compositions to reduce the odor of odor bearing materials.

### DETAILED DESCRIPTION OF THE INVENTION

The compositions of the present invention are useful for reducing undesirable odors on materials. The materials are applied in aqueous form, such as by spraying from a container, to cause the composition to interact with the odor molecules and reduce their detectable presence. Thus, the products of the present invention have, as a primary mechanism for reducing odor, the ability to interact with odor molecules and reduce their detectable presence in the environment.

The preferred odor counteractant components are aldehyde-containing compositions which may also optionally include some fragrance component. Suitable counteractant compositions are described in US patents 5,795,566 and 4,840,792.

The preferred counteractant components useful in the present invention comprise combinations of aldehydes which have been found to be superior to the compounds when used individually. The compositions comprise a first aldehyde chosen from the acyclic and non-terpenic aliphatic aldehydes the non-terpenic alicyclic aldehydes, the terpenic aldehydes, the aliphatic aldehydes substituted by an aromatic group and the bifunctional aldehydes (for convenience all designated as"group A"aldehydes). The second type of aldehyde (for convenience designated as"group B"aldehydes) are selected for the aldehydes possessing a non-aromatic unsaturation carried by the carbon in the alpha position of the aldehyde function, the aldehydes possessing an unsaturation in the alpha position of the aldehyde function conjugated with an aromatic ring and the aldehydes of which the function is carried by an aromatic ring. A number of examples of each group are recited in the aforementioned US patents. The compositions contain at least one aldehyde form each of the above groups and can contain three or more aldehydes as long as there is at least one from each of the groups noted above. These materials are available from Robertet Fragrances of Oakland, New Jersey, and are available as alcohol-free compositions with varying notes and amounts of fragrance component or may be fragrance-free. These Robertet products are designated as TQ19, TE-77, UC 11 and various other designations.

It is particularly preferred that the compounds according to the present invention be chosen to have a relatively low vapor pressure so that they remain on the surface to be treated for a reasonable period of time. Generally, compounds having a vapor pressure equal to or less than about 4 Pascals at 25 degrees centigrade are preferred.

The aldehyde pairs of groups A and B can be in relative proportions one to the other and preferably in proportions from about 80/20 to 20/80 and particularly in proportions of about equal amounts.

Some combinations of the aldehydes have fragrance properties of their own and may serve to mask odors as well as to counteract them by reaction with and suppression of the odor molecules. Fragrant masking components can also be added to the counteractant compositions to provide a masking effect, if desired. In addition, the counteractant component can be formulated so that there is little or no detectable fragrance component which may be desirable for certain environments.

The counteractant component is preferably solubilized in an aqueous vehicle to form a concentrate containing from about 10% to about 50% of the counteractant by weight of the total composition and most preferably about 25% by weight of the composition. This composition can be diluted with about 1 part by weight of the concentrate to about 75 parts or more by weight water so that the counteractant component is present in the ready to use form in an amount of from about 0.1 % to about 1% by weight of the total composition.

The aldehyde-containing counteractant component of the present invention is not readily soluble in water. Therefore solubilizing agents must be used to permit these types of materials to be dispersed or solubilized in aqueous vehicles. Various surfactant materials are well known in the art as solubilizing agents. However, it has been found by the present inventors that the commonly used surfactants when used alone are not suitable to prepare the clear, stable concentrates of the present invention. In the present case a further co-solvent is needed in combination with one or more surfactants to help solubilize the counteractant to provide a concentrate which is stable for storage and can be diluted to a clear solution for use in the present invention.

The surfactants that have been found most useful in the present invention are the nonionic surfactants. Some anionic surfactants are not preferred because they may have greater capability to form insoluble products with the surfaces to which applied or other ingredients in the counteractant composition and thereby leave deposits on the surface which could, in some cases be visible to an observer. The useful surfactants can comprise one or more low molecular weight polymers, such as the alcohol ethoxylates or polyethylene glycol ethers, for example alkyloxypolyethyleneoxyethanol, available from Union Carbide under the "Tergitol" brand or from Shell Oil Company under the "Neodol" brand.

As noted above, co-solvents are necessary to enable the counteractant component to be solubilized satisfactorily in water at the relatively high concentrations needed for providing dilutable, commercial quality concentrates without gelling. The preferred cosolvents for use in the present invention are the glycol ethers, such as diethylene glycol ethyl ether available under the trade name "Carbitol" from the Union Carbide Corporation.

Generally the co-solvent is present in the concentrate in an amount of about 2 to about 20 percent by weight and most preferably about 10 percent by weight.

Other ingredients may optionally be added to the composition without altering its effect as an odor counteractant. For example an acid may be added to control the pH of the composition to be slightly acidic so that the counteractant will more readily react with amine-based malodor molecules and suppress their odor. Citric acid is useful to control the pH of the composition. Citric acid has the added advantage of being a counteractant against certain amine or urea odors such as found in urine.

A preservative may also be added to keep bacteria or molds from growing during storage or after application. The preservative should be chosen so that it does not react with the counteractant component and reduce its effectiveness. A variety of preservative materials can be used such as 1, 2-Benzisothiazolin-3-one, available commercially as a solution in dipropylene glycol as Proxel GXL from Zeneca AG Products, Inc.

In order to prepare odor counteractant compositions that have sanitizing, disinfecting or biocidal properties, the present inventors have found that certain quaternary amine disinfectant compositions known to have biocidal properties can be effective in the present invention, namely N-alkyl, N-dimethylarnmonium chloride and didecyl dimethylammonium chloride. These compounds impart disinfecting capabilities to the compositions of the present invention without significantly reducing the counteractant effects of the compositions. These materials are available from Lonza, Inc., Fairlawn, New Jersey under the trade designations of BTC 1010 and Bardac 2250, respectively. Other quaternary amines and other known biocidal components, such as certain biguanides, have not been shown to be effective in the compositions of the present invention.

Generally the useful compounds are effective when present in a range of 0.05 to about 5.0 percent by weight of the composition in the diluted form. Because the materials are often diluted from 25 to 150 times and more preferably from 60 to 75 times, the concentrate will generally have from about 2 to about 20 percent by weight of the quaternary amine and preferably about 5 to 10% by weight of the concentrate. Tests of the compositions of the present invention, as shown in the Example below, have demonstrated efficacy against staphylococcus aureus.

The compositions of the present invention can be prepared by simple mixing of the ingredients using liquid bulk mixers well known in the art, particularly rotating mixers of various types. The ingredients can be mixed in any particular order that is compatible with the equipment being used and which provides a stable composition. Certain of the ingredients, e. g. the castor oil, may be solid at room temperature, but can be heated to the molten state and then combined as a liquid with the other ingredients.

The diluted compositions can be applied by a variety of methods well known in the art. Generally the diluted compositions will be applied from a hand trigger spray bottle commonly used for household and commercial cleaning products. In a short time after application, the compositions are effective to reduce the odor impression as tested by quantitative and qualitative techniques, such as sniff tests. Moreover, the compositions have shown a superior ability to maintain the odor reduction longer than competitive products, particularly for certain types of odors, such as tobacco odor. For example, the compositions of the present invention have demonstrated the ability to reduce odor impressions for up to three days or more.

The present invention can be illustrated by reference to the following example, wherein all compositions are in percent by weight unless otherwise indicated.

### Example 1 (not according to the invention)

Diluted samples of the odor counteractant, solubilizing surfactant and water were prepared for efficacy testing comprising:

| | |
|---|---|
| Aldehyde counteractant (Robertet TE-77) | 0.39 wt. % |
| Ethoxylated linear alcohol (Tergitol 15-s-9) | 0.92 wt. % |
| Dl water | 98.69 wt. % |

The above composition was tested against other commercially available counteractant compositions for counteracting various odors. In one test, fabric that had absorbed smoke odor in a smoke chamber was sprayed front and back with the composition until damp. The fabric swatches were then placed in 16 ounce (450 g) glass jars and covered and allowed to sit for 5 minutes. After 5 minutes the jars were opened and 14 participants sniffed the samples to determine the least amount of smoke malodor present. Of the 14 participants, 9 chose the fabric samples treated with the formula shown above.

In another test, 75 ml of the liquid formula shown above was placed in 16 ounce (450 g) glass jars and gaseous samples of several malodorous chemicals representative of various household odors were pipetted into the liquid and allowed the samples to sit white the jars were sealed. The jars were opened and the head space above the liquid sampled with a photoionization detector to detect the malodor concentration in ppm. The same test was run with various competitive products and with water and compared. Using the water as a standard. The results are shown in the table below as the average percent reduction in malodor concentration in the headspace of the jar after sitting for 1 minute, 5 minutes and 10 minutes compared with water as the standard.

| Malodor | Formula | 1 minute | 5 minutes | 10 minutes |
|---|---|---|---|---|
| Methyl trisulfide | Formula 1 | 97.66 | 88.97 | 76.04 |
| | Competitive A | 87.58 | 85.23 | 66.55 |
| | Competitive B | 94.51 | 84.10 | 58.57 |
| Triethylamine | Formula 1 | 58.4 | 59.96 | 49.29 |
| | Competitive A | 85.59 | 88.08 | 88.67 |
| | Competitive B | 63.17 | 66.95 | 60.03 |
| 1-Hexanethiol | Formula 1 | 62.93 | 46.18 | 38.39 |
| | Competitive A | 62.77 | 43.23 | 39.36 |
| | Competitive B | 67.79 | 52.46 | 46.76 |
| Methyldisulfide | Formula 1 | 96.36 | 88.85 | 81.35 |
| | Competitive A | 18.37 | 22.42 | 38.23 |
| | Competitive B | 91.86 | 79.89 | 67.87 |

The composition of the present invention was as effective or better than the competitive products on most malodors. It did not perform as well as one competitor on triethylamine which is representative of fishy malodors.

### Example 2 (not according to the invention)

Concentrates of the compositions similar to those of the dilute formula stated above were prepared by combining the same ingredients at higher concentrations of the aldehyde counteractant and surfactant. However, the compositions were cloudy and not suitable for commercial use without subsequent processing. The use of the solubilizing agents and co-solvents according to the present invention permitted the concentrated compositions to be prepared as shown below:

| | |
|---|---|
| Ethoxylated Linear Alcohol (Tergitol 15-s-9) | 48.34% |
| Aldehyde counteractant* | 10.0 |
| Hydrogenated Castor Oil (PEG 40) | 25.0 % |
| Diethylene Glycol Monoethyl Ether (Carbitol) | 10.0 % |
| Citric Acid, 50% liquid | 1.4 % |
| 1, 2-Benzisothiazolin-3-one, 10% (Proxel GXL) | 0.06% |
| Deionized water | 5.2 % |

| | |
|---|---|
| * The aldehyde counteractant used in the various formulas were Robertet dual aldehyde counteractants which each contained different fragrance notes and amounts and are identified by Robertet as TE-77, TQ-19, UC 11, etc. | |

The formulas above formed clear, stable liquid mixtures that were diluted 60 or 75 times with water to provide a clear, sprayable liquid odor counteractant for application to various surfaces. When tested on various malodors, such as tobacco smoke, onion and garlic, these compositions provided the counteractant effects comparable to those shown in the table above. Moreover, these materials provided a counteractant effect that persisted for several hours longer than the competitive products against which it was tested.

### Example 3 according to the invention

The following compositions demonstrate the ability to impart disinfecting characteristics to the odor counteractant compositions of the present invention using a select group of quaternary amine compounds.

| | A | B | C | D | E |
|---|---|---|---|---|---|
| Ethoxylated Linear Alcohol (Neodol 91-8) | 48.0 | 48.0 | 48.0 | 48.0 | 48.0 |
| Aldehyde counteractant | 9.0 | 9.0 | 9.0 | 9.0 | 9.0 |
| Diethylene Glycol Monoethyl Ether (Carbitol) | 7.8 | 7.8 | 7.8 | 7.8 | 7.8 |
| Citric Acid, 50% liquid | 1.2 | 1.2 | 1.2 | 1.2 | 1.2 |
| Deionized water | 21.3 | 22.0 | 22.0 | 28.0 | 28.0 |
| Quaternary amine (BTC 1010) | 7.5 | -- | -- | -- | -- |
| Quaternary amine (Bardac 2250) | -- | 12.0 | -- | 6.0 | -- |
| Quaternary amine (Cation G 50) (comparative) | -- | -- | 12.0 | -- | 6. 0 |
| AOAC* Test results (diluted): | 0/10 | 1/10 | 2/10 | 1/10 | 3/10 |

| | | | | | |
|---|---|---|---|---|---|
| * Association of Official Analytical Chemists test for germicidal spray product as disinfectants, "Official Methods of Analysis of AOAC International", 16^{th} Edition, Section 961.02, copyright 1998) | | | | | |

The compositions were diluted with water at a ratio of 60: 1 and tested in accordance with the AOAC Germicidal test with a contact time of 5 minutes. As shown above, formula A was superior demonstrating no growth of bacteria in all 10 of the test cylinders, formulas B and D were acceptable showing growth of bacteria in only 1 of the 10 cylinders and formulas C and E were not acceptable showing bacteria growth in 2 and 3 of the 10 cylinders respectively.

## Claims

1. A clear, stable, acidic, alcohol-free concentrate for reducing the odor of odor-bearing inanimate materials when diluted from 25 to 150 times with water, the concentrate comprising
(a) from 10 to 50 percent by weight of the total composition of an aldehyde-based odor counteractant component;
(b) from 25 to 60 percent by weight of the total composition of a solubilizing agent for the odor counteractant component;
(c) from 2 to 20 percent by weight of the total composition of a co-solvent for the odor counteractant component; and
(d) from 2 to 20 percent by weight of the total composition of a compatible quaternary amine disinfectant selected from the group consisting of N-alkyl, N-dimethyl ammonium chloride and didecyl dimethylammonium chloride or mixtures thereof, which does not degrade the performance of the odor counteractant component;
(e) the remainder being an aqueous vehicle.

2. The concentrate according to claim 1 wherein the concentrate additionally comprises an acidic pH control agent.

3. The concentrate according to claim 1 wherein the concentrate additionally comprises a fragrance component.

4. The concentrate according to claim 1 wherein the concentrate additionally comprises a preservative.

5. The concentrate according to claim 1 wherein the odor counteractant component comprises a combination of aldehyde compounds comprising
at least one first aldehyde selected from the group consisting of acyclic and non-terpenic aliphatic aldehydes, non-terpenic alicyclic aldehydes, terpenic aldehydes, aliphatic aldehydes substituted by an aromatic group, and bifunctional aldehydes and
at least one second aldehyde selected from the group consisting of aldehydes possessing a non-aromatic unsaturation carried by the carbon in the alpha position of the aldehyde function, aldehydes possessing an unsaturation in the alpha position of the aldehyde function conjugated with an aromatic ring, and aldehydes of which the function is carried by an aromatic ring.

6. The concentrate according to claim 1 wherein the odor counteractant component comprises at least one aldehyde compound having a vapor pressure no greater than about 4 Pascals at 25 degrees centigrade.

7. The concentrate according to claim 1 wherein the solubilizing agent is alkyloxypolyethyleneoxyethanol.

8. The concentrate according to claim 1 wherein the co-solvent Is a glycol ether.

9. A liquid compositon for applying to the surface of an odor-bearing inanimate material without further dilution, the composition comprising
(a) from 0.1 to 1.0 percent by weight of the total composition of an aldehyde-based odor counteractant component;
(b) from 0.3 to 0.8 percent by weight of the total composition of a solubilizing agent for the counteractant component;
(c) from 0.03 to 0.3 percent by weight of the total composition of a co-solvent for the counteractant component; and
(d) from 0.05 to 5.0 percent by weight of a compatible quaternary amine disinfectant selected from the group consisting of N-alkyl, N-dimethyl ammonium chloride and didecyl dimethylammonium chloride or mixtures thereof, which does not degrade the performance of the odor counteractant component;
(e) the remainder being an aqueous vehicle.

10. The liquid composition according to claim 9 wherein said co-solvent is a glycol ether.

11. A method of counteracting odors **characterized i**n that it comprises
(a) preparing a clear, stable, acidic, alcohol-free concentrate according to claim 1;
(b) diluting the composition to at least about 25 times with water; and
(c) applying the diluted composition onto an odor-bearing surface.

12. The method according to claim 11 wherein the composition is diluted between 25 to 150 times with water.

## Patentansprüche

1. Klares, stabiles, saures, alkoholfreies Konzentrat zur Verminderung des Geruchs von geruchsbehafteten, nicht-lebenden Materialien beim Verdünnen auf das 25- bis 150-fache mit Wasser, wobei das Konzentrat umfasst
(a) 10 bis 50 Gewichtsprozent der Gesamtzusammensetzung einer aldehydbasierten geruchsneutralisierenden Komponente;
(b) 25 bis 60 Gewichtsprozent der Gesamtzusammensetzung eines Lösungsvermittlers für die geruchsneutralisierende Komponente;
(c) 2 bis 20 Gewichtsprozent der Gesamtzusammensetzung eines Hilfslösungsmittels für die geruchsneutralisierende Komponente; und
(d) 2 bis 20 Gewichtsprozent der Gesamtzusammensetzung eines kompatiblen Desinfektionsmittels aus quartärem Amin, ausgewählt aus der Gruppe, die aus N-Alkyl,N-dimethylammoniumchlorid und Didecyldimethylammoniumchlorid oder deren Gemischen besteht, das die Leistungsfähigkeit der geruchsneutralisierenden Komponente nicht beeinträchtigt;
(e) wobei der Rest ein wässriger Träger ist.

2. Konzentrat nach Anspruch 1, worin das Konzentrat zusätzlich ein saures pH-Einstellmittel enthält.

3. Konzentrat nach Anspruch 1, worin das Konzentrat zusätzlich eine Duftstoffkomponente enthält.

4. Konzentrat nach Anspruch 1, worin das Konzentrat zusätzlich einen Konservierungsstoff enthält.

5. Konzentrat nach Anspruch 1, worin die geruchsneutralisierende Komponente eine Kombination aus Aldehydverbindungen umfasst, umfassend mindestens einen ersten Aldehyd, der aus der Gruppe ausgewählt ist, die aus acyclischen und nicht-terpenischen aliphatischen Aldehyden, nicht-terpenischen alicyclischen Aldehyden, terpenischen Aldehyden, aliphatischen Aldehyden, substituiert mit einer aromatischen Gruppe, und bifunktionellen Aldehyden besteht, und mindestens einem zweiten Aldehyd, ausgewählt aus der Gruppe, die aus Aldehyden mit einer nicht-aromatischen Ungesättigtheit, die von dem Kohlenstoff in der alpha-Position der Aldehydfunktion getragen wird, Aldehyden mit einer Ungesättigtheit in der alpha-Position der Aldehydfunktion, konjugiert mit einem aromatischen Ring, und Aldehyden, deren Funktionen durch einen aromatischen Ring getragen werden, besteht.

6. Konzentrat nach Anspruch 1, worin die geruchsneutralisierende Komponente mindestens eine Aldehydverbindung mit einem Dampfdruck von nicht mehr als etwa 4 Pascal bei 25 Grad Celsius umfasst.

7. Konzentrat nach Anspruch 1, worin der Lösungsvermittler Alkyloxypolyethylenoxyethanol ist.

8. Konzentrat nach Anspruch 1, worin das Hilfslösungsmittel ein Glycolether ist.

9. Flüssige Zusammensetzung zum Aufbringen auf die Oberfläche eines geruchsbehafteten, nicht-lebenden Materials ohne weitere Verdünnung, wobei die Zusammensetzung umfasst
(a) 0,1 bis 1,0 Gewichtsprozent der Gesamtzusammensetzung einer aldehydbasierten, geruchsneutralisierenden Komponente;
(b) 0,3 bis 0,8 Gewichtsprozent der Gesamtzusammensetzung eines Lösungsvermittlers für die neutralisierende Komponente;
(c) 0,03 bis 0,3 Gewichtsprozent der Gesamtzusammensetzung eines Hilfslösungsmittels für die neutralisierende Komponente; und
(d) 0,05 bis 5,0 Gewichtsprozent eines kompatiblen Desinfektionsmittels aus quartärem Amin, ausgewählt aus der Gruppe, die aus N-Alkyl,N-dimethylammoniumchlorid und Didecyldimethylammoniumchlorid oder deren Gemischen besteht, welches die Leistungsfähigkeit der geruchsneutralisierenden Komponente nicht beeinträchtigt;
(e) wobei der Rest ein wässriger Träger ist.

10. Die flüssige Zusammensetzung gemäß Anspruch 9, worin das Hilfslösungsmittel ein Glycolether ist.

11. Verfahren zum Neutralisieren von Gerüchen, **dadurch gekennzeichnet, dass** es umfasst:
(a) Herstellen eines klaren, stabilen, sauren, alkoholfreien Konzentrats gemäß Anspruch 1;
(b) Verdünnen der Zusammensetzung auf mindestens etwa das 25-fache mit Wasser; und
(c) Aufbringen der verdünnten Zusammensetzung auf eine geruchsbehaftete Oberfläche.

12. Verfahren nach Anspruch 11, worin die Zusammensetzung zwischen 25-bis 150-fach mit Wasser verdünnt wird.

## Revendications

1. Concentré sans alcool, acide, stable et limpide servant à atténuer les odeurs de matériaux inanimés odorants une fois dilué de 25 à 150 fois avec de l'eau, le concentré comprenant
(a) de 10 à 50 pour cent en poids de la composition totale d'un composant neutralisant les odeurs à base d'aldéhydes ;
(b) de 25 à 60 pour cent en poids de la composition totale d'un agent de solubilisation pour le composant neutralisant les odeurs ;
(c) de 2 à 20 pour cent en poids de la composition totale d'un co-solvant pour le composant neutralisant les odeurs ; et
(d) de 2 à 20 pour cent en poids de la composition totale d'un désinfectant à base d'amine quaternaire compatible choisi dans le groupe constitué du chlorure de N-alkyl,N-diméthylammonium et du chlorure de didécyldiméthylammonium ou de leurs mélanges, qui ne détériore pas les performances du composant neutralisant les odeurs ;
(e) le reste étant un véhicule aqueux.

2. Concentré selon la revendication 1, lequel concentré comprend en outre un agent de régulation du pH acide.

3. Concentré selon la revendication 1, lequel concentré comprend en outre un composant de type parfum.

4. Concentré selon la revendication 1, lequel concentré comprend en outre un conservateur.

5. Concentré selon la revendication 1, dans lequel le composant neutralisant les odeurs comprend une combinaison de composés aldéhydes comprenant au moins un premier aldéhyde choisi dans le groupe constitué des aldéhydes acycliques et aliphatiques non terpéniques, des aldéhydes alicycliques non terpéniques, des aldéhydes terpéniques, des aldéhydes aliphatiques substitués par un groupe aromatique, et des aldéhydes bifonctionnels et au moins un deuxième aldéhyde choisi dans le groupe constitué des aldéhydes possédant une insaturation non aromatique portée par le carbone en position alpha de la fonction aldéhyde conjuguée avec un cycle aromatique, et des aldéhydes dont la fonction est portée par un cycle aromatique.

6. Concentré selon la revendication 1, dans lequel le composant neutralisant les odeurs comprend au moins un composé aldéhyde ayant une tension de vapeur ne dépassant pas environ 4 Pascals à 25 degrés centigrade.

7. Concentré selon la revendication 1, dans lequel l'agent de solubilisation est l'alkyloxypolyéthylèneoxyéthanol.

8. Concentré selon la revendication 1, dans lequel le co-solvant est un éther de glycol.

9. Composition liquide à déposer à la surface d'un matériau inanimé odorant sans dilution supplémentaire, la composition comprenant
(a) de 0,1 à 1,0 pour cent en poids de la composition totale d'un composant neutralisant les odeurs à base d'aldéhydes ;
(b) de 0,3 à 0,8 pour cent en poids de la composition totale d'un agent de solubilisation pour le composant neutralisant les odeurs ;
(c) de 0,03 à 0,3 pour cent en poids de la composition totale d'un co-solvant pour le composant neutralisant les odeurs ; et
(d) de 0,05 à 5,0 pour cent en poids d'un désinfectant à base d'amine quaternaire compatible choisi dans le groupe constitué du chlorure de N-alkyl,N-diméthylammonium et du chlorure de didécyldiméthylammonium ou de leurs mélanges, qui ne détériore pas les performances du composant neutralisant les odeurs ;
(e) le reste étant un véhicule aqueux.

10. Composition liquide selon la revendication 9, dans laquelle ledit co-solvant est un éther de glycol.

11. Procédé de neutralisation d'odeurs **caractérisé en ce qu'**il comprend
(a) la préparation d'un concentré sans alcool, acide, stable et limpide selon la revendication 1 ;
(b) la dilution de la composition au moins environ 25 fois avec de l'eau ; et
(c) le dépôt de la composition diluée sur une surface odorante.

12. Procédé selon la revendication 11, dans lequel la composition est diluée entre 25 et 150 fois avec de l'eau.
